(19) Europäisches Patentamt
European Patent Office
Office européen des brevets

(11) **EP 2 177 539 A1**

(12) **EUROPEAN PATENT APPLICATION**
published in accordance with Art. 153(4) EPC

(43) Date of publication:
**21.04.2010 Bulletin 2010/16**

(21) Application number: **08790343.1**

(22) Date of filing: **01.08.2008**

(51) Int Cl.:
*C08B 37/08* (2006.01)     *A61K 8/60* (2006.01)
*A61K 8/73* (2006.01)     *A61K 47/36* (2006.01)
*A61Q 1/00* (2006.01)     *A61Q 5/00* (2006.01)
*A61Q 19/00* (2006.01)     *A61Q 19/02* (2006.01)
*B01F 17/56* (2006.01)

(86) International application number:
**PCT/JP2008/002079**

(87) International publication number:
**WO 2009/019840 (12.02.2009 Gazette 2009/07)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **09.08.2007 JP 2007208503**

(71) Applicant: **Kibun Food Chemifa Co., Ltd.**
**Tokyo 1048553 (JP)**

(72) Inventors:
• **HIRAI, Hideki**
  **Tokyo 104-8553 (JP)**
• **WATANABE, Makoto**
  **Tokyo 104-8553 (JP)**
• **NOZAWA, Takashi**
  **Tokyo 104-8553 (JP)**
• **MURATA, Katsumi**
  **Tokyo 104-8553 (JP)**

(74) Representative: **Zimmermann & Partner**
**Postfach 330 920**
**80069 München (DE)**

(54) **PROPYLENE GLYCOL HYALURONATE ESTER AND EXTERNAL PREPARATION FOR SKIN USING THE SAME**

(57) A propylene glycol hyaluronate having an esterification degree of from 10 to 90% and a limiting viscosity of less than 3 dL/g has an excellent percutaneous absorption-promoting effect, and is widely usable as cosmetics and pharmaceutical products.

**Description**

TECHNICAL FIELD

The present invention relates to a propylene glycol hyaluronate satisfying a specific condition, and to a skincare preparation for external use comprising the propylene glycol hyaluronate. The skincare preparation for external use of the invention has a percutaneous absorption-promoting effect for active substances, and has various excellent **characteristics in that** it is poorly stringy and is readily soluble in an alcohol solvent but hardly flows away with sweat, it well emulsifies and when applied to a skin, its feel is good. Accordingly, it is widely usable as cosmetics and pharmaceutical products.

BACKGROUND ART

Skin trouble is caused by excessive loss of moisture from the surface of the skin exposed to dry air or cleansing. In these days, various chemical substances flood in the environment, and the skin exposed to such chemical substances may lose its functions, often bringing about skin trouble owing to lipid secretion depression. Therefore, there is a demand for a skincare preparation for external use capable of preventing skin trouble and capable of providing an excellent moisturizing effect.

Various active compounds having a moisturizing effect have been provided, such as typically water-soluble polyols. Some of them have already been commercialized, such as propylene glycol. However, many of commercialized moisturizing compounds are associated with uncomfortable feel during application or insufficient moisturizing effect, so that there is still a demand for the development of new moisturizing compounds.

Another moisturizing compound is sodium hyaluronate, which draws special interest as a valuable compound because of high hydration effect. However, aqueous solutions of sodium hyaluronate have a disadvantage in stability, which is high within a neutral pH range but lowers in acidic solutions or salt solutions. This leads to the problem that the compound could not effectively produce a moisturizing effect under some storage conditions or application conditions during use as cosmetics or the like, and there is a need for a solution thereto.

As opposed to this, a propylene glycol hyaluronate satisfying a condition that its limiting viscosity is at least 3 dL/g (about 100,000 in terms of its molecular weight) is provided as a moisturizing compound having an excellent viscosity stability in a low-pH system or a cation-existing system (see Patent Reference 1). According to Examples in this reference, it has become clarified that the ester can effectively exhibit its moisturizing property irrespective of storage conditions or application conditions in use.

Patent Reference 1: JP-A 2001-233901

DISCLOSURE OF THE INVENTION

PROBLEMS THAT THE INVENTION IS TO SOLVE

On the other hand, it is generally known that a water-soluble compound such as arbutin having a skin-whitening effect is hardly percutaneously absorbable. For use as cosmetics, a water-soluble compound is desired to penetrate into the depth of the skin to which it is applied, and the compound is preferably combined with a compound having a percutaneous absorption-promoting effect. However, at present, no one has an information capable of giving any predictability in that what compound could have a percutaneous absorption-promoting effect.

Give that situation, the present inventors have investigated various compounds in point of their percutaneous absorption-promoting effect and, as a result, have found that many of those heretofore proposed as useful compounds for cosmetic applications could not always have a sufficient percutaneous absorption-promoting effect. For example, sodium hyaluronate and propylene glycol hyaluronate satisfying the condition that its limiting viscosity is at least 3 dL/g disclosed in Patent Reference 1 were analyzed for their percutaneous absorption-promoting effect, which, however, confirmed that their percutaneous absorption-promoting effect is not sufficiently satisfactory.

Taking the above-mentioned prior-art problems into consideration, the present inventors have further investigated for the purpose of providing a compound having an excellent percutaneous absorption-promoting effect and capable of being used widely in cosmetics and pharmaceutical products. In particular, the inventors have further promoted the investigation for the purpose of providing a compound which, in addition to the usefulness mentioned in the above, has other advantages in that it is poorly stringy but is readily soluble in an alcohol solvent, and that, on the other hand, it hardly flows away with sweat and well emulsifies and, when applied to a skin, gives a good feel thereto, and providing a skincare preparation for external use.

## MEANS FOR SOLVING THE PROBLEMS

The inventors have assiduously studied for the purpose of solving the above-mentioned problems and, as a result, have found that a propylene glycol hyaluronate satisfying a specific condition has an excellent property, and have completed the present invention.

Specifically, the invention provides a propylene glycol hyaluronate having an esterification degree of from 10 to 90%, preferably from 20 to 80%, more preferably from 25 to 73%. The invention also provides a propylene glycol hyaluronate having a limiting viscosity of less than 3 dL/g, preferably from 0.2 to 2.8 dL/g, more preferably from 0.3 to 2.3 dL/g, even more preferably from 0.3 to 2.0 dL/g, still more preferably from 0.4 to 1.8 dL/g, further more preferably from 0.4 to 1.6 dL/g, especially more preferably from 0.5 to 1.2 dL/g. Especially preferably, the propylene glycol hyaluronate of the invention is a compound having an esterification degree of from 25 to 73% and a limiting viscosity of from 0.5 to 1.2 dL/g. Preferably, the propylene glycol hyaluronate of the invention is combined with a cosmetic substance or a pharmaceutically active compound (e.g., a skin-whitening active compound).

The invention also provides a skincare preparation for external use which comprises the above-mentioned propylene glycol hyaluronate. The skincare preparation for external use of the invention is useful as a moisturizing agent, an emulsifying agent, a skin-whitening agent, etc. In this description, the numerical range expressed by the wording "a number to another number" means the range that falls between the former number indicating the lowermost limit of the range and the latter number indicating the uppermost limit thereof.

## EFFECT OF THE INVENTION

The propylene glycol hyaluronate of the invention has characteristic advantages in that it has a percutaneous absorption-promoting effect for active substances, its effect of keeping water in a horny layer is high, it is poorly stringy, it is readily soluble in an alcohol solvent but hardly flows away with sweat, it viscosity stability in a broad pH range is excellent, its emulsifiability is good, and when applied to a skin, its feel is good. Accordingly, a skincare preparation for external use comprising the propylene glycol hyaluronate of the invention is especially useful as a component of cosmetics and pharmaceutical products.

## MODE FOR CARRYING OUT THE INVENTION

The propylene glycol hyaluronate and the skincare preparation for external use of the invention are described in detail hereinunder. The description of the constitutive elements of the invention given hereinunder is for some typical embodiments of the invention, to which, however, the invention should not be limited.

The propylene glycol hyaluronate of the invention has an esterification degree of from 10 to 90%, and a limiting viscosity of less than 3 dL/g. Preferred ranges of the esterification degree and the limiting viscosity are as mentioned in the above. Preferably, the propylene glycol hyaluronate of the invention is a compound having an esterification degree of from 10 to 90% and a limiting viscosity of from 0.3 to 2.0 dL/g. More preferred is a compound having an esterification degree of from 10 to 90% and a limiting viscosity of from 0.4 to 1.6 dL/g. Even more preferred is a compound having an esterification degree of from 10 to 90% and a limiting viscosity of from 0.5 to 1.2 dL/g.

In this description, the "esterification degree" means a ratio of the esterified one in the carboxylic acid that constitutes hyaluronic acid. The limiting viscosity of the propylene glycol hyaluronate is measured in a 0.2 mol/L sodium chloride solution at a temperature of 25˚C.

As compared with hyaluronic acid and hyaluronic acid salts (e.g., sodium salt) having a molecular weight on the same level, the propylene glycol hyaluronate of the invention has a more excellent viscosity stability in a broad pH range and is poorly stringy, it is excellent in point of the moisturizing effect, the percutaneous absorption-promoting effect and the effect of keeping water in a horny layer, and it is resistant to enzymes such as hyaluronidase, etc. In addition, its solubility in cationic substances (especially in cationic surfactants) is high, and therefore the ester has another advantage in that it is useful in producing commercial products of rinses.

The hyaluronic acid moiety that constitutes the propylene glycol hyaluronate of the invention is not specifically defined in point of its type and structure. Hyaluronic acid is a polysaccharide comprising a biose of D-glucuronic acid and N-acetyl-D-glucosamine as a repetitive unit, and its molecular weight is not specifically defined so far as the limiting viscosity of its propylene glycol ester falls within the above-mentioned range. Hyaluronic acid for use in the invention may be a synthetic one, or one purified from a natural substance in a known method. D- glucuronic acid and N-acetyl-D-glucosamine that constitute hyaluronic acid may be partly substituted within a range not detracting from the effect of the invention. For example, the hydroxyl group may be substituted to form an alkoxy group. The substitution may be attained within a range obvious to those skilled in the art. In the propylene glycol hyaluronate of the invention, the acid moiety and the ester moiety may be localized in the molecule, or may be uniformly dispersed therein. However, a case of two or more hyaluronic acid molecules crosslinked with propylene glycol shall be excluded.

The method for producing the propylene glycol hyaluronate of the invention is not specifically defined. Especially preferred is a method of reacting a mixture of hyaluronic acid and sodium hyaluronate with propylene oxide. Concretely, first, sodium hyaluronate is partly converted into hyaluronic acid with a hydrochloric acid/ethanol solution or the like, then heated to reduce the molecular weight thereof, and then washed with ethanol. As a result, a mixture of hyaluronic acid and sodium hyaluronate is produced. Next, propylene oxide is added to the mixture and heated for esterification. The esterification temperature is preferably from 50 to 80˚C; and the reaction time is preferably from 0.1 to 4 hours or so. After the reaction, this may be washed with ethanol, neutralized with an ethanol solution of sodium acetate, again washed with ethanol and then dried. According to the production method, the propylene glycol hyaluronate of the invention having the desired effect can be produced efficiently.

The propylene glycol hyaluronate of the invention is extremely useful as a constitutive ingredient of a skincare preparation for external use. The propylene glycol hyaluronate of the invention is has a good viscosity stability, and is effective for emulsification, moisturization, percutaneous absorbability promotion and water retentiveness in horny layer. Accordingly, the skincare preparation for external use comprising the propylene glycol hyaluronate of the invention is useful as a moisturizing agent, an emulsifier, a percutaneous absorption promoter, etc.

The skincare preparation for external use of the invention is, for example, as combined with a skin whitening agent such as arbutin, applied to the surface of a skin to promote the penetration of the skin-whitening agent into the inside of the skin to thereby stably exhibit the excellent skin-whitening effect of the agent. The excellent effect is noticeably recognized in the propylene glycol hyaluronate that satisfies the above-mentioned limiting viscosity condition in the invention. The fact that the effect of the propylene glycol hyaluronate satisfying the above-mentioned limiting viscosity condition is noticeably higher than that of any other propylene glycol hyaluronate not satisfying the above-mentioned limiting viscosity condition could not be predicted at all from conventional studies.

The compound that may be combined with the propylene glycol hyaluronate in the skincare preparation for external use of the invention is not limited to arbutin. Any other skin-whitening agents except arbutin, as well as kojic acid, ascorbic acid derivatives and other many cosmetically or pharmaceutically active compounds such as those mentioned below can be combined with it. In particular, the ester is preferably combined with a water-soluble compound.

The skincare preparation for external use of the invention has a moisturizing effect, and therefore can suitably moisturize and smooth the surface of a skin to which it has been applied. Further, the skincare preparation for external use of the invention is **characterized in that** it is poorly stringy, and therefore its advantages are that it is easy to use and its feel is good. In particular, when applied to a skin, the preparation can make the skin fresh and young, and its users can experience good spreading of the preparation on their skin.
Further, the skincare preparation for external use of the invention is **characterized in that** its solubility in alcohol such as ethanol and glycol is high. Accordingly, especially in the filed of cosmetics, the preparation is excellent in the processability and the handlability, and enables its wide range applications. Further, the skincare application for external use of the invention is **characterized in that** it relatively hardly flows away with water, and its another advantage is that it hardly flows away with sweat when applied to a skin.

The skincare preparation for external use of the invention can be effectively used as cosmetics and pharmaceutical products. For example, it can be used as toilet soap, shampoo, face wash, rinse, eye cream, eye shadow, cream, milky liquid, lotion, perfume, face powder, cosmetic oil, hair cosmetics, hair dye, perfume paste, powder, pack, shaving cream, shaving lotion, suntan oil, sunshield oil, suntan lotion, sunshield lotion, suntan cream, sunshield cream, foundation, powdery perfume, cheek rouge, mascara, eyebrow pencil, nail cream, nail enamel, nail enamel remover, hair wash, bath cosmetics, lipstick, lip cream, eye liner, tooth paste, deodorant, cologne, hair tonic, hair restorer, etc. In addition, the skincare preparation for external use of the invention is usable also as ointment, poultice, etc.

The skincare preparation for external use of the invention may further comprise various ingredients except propylene glycol hyaluronate added thereto in accordance with its use and object. For example, suitable ingredients may be added to it for the purpose of emollient effect enhancement, feel improvement, roughness reduction after use, solubility enhancement, emulsifiability enhancement, emulsion stability enhancement, compatibility enhancement with oily ingredients, skin stiffness reduction after use, compatibility enhancement with skin, spreadability enhancement on skin, stickiness reduction, skin trouble prevention, skin-aesthetic effect enhancement, skin-protective effect enhancement, cuticle refreshment, epidermal keratinization normalization (prevention of parakeratosis owing to skin turnover intensification, epidermal hyperplasia prevention, epidermal dyslipidemia prevention), care of xeroderma such as senile xeroderma, care of dry skin such as skin cracking or scaling, wrinkling prevention, wrinkle removal, wound care, prevention and remedy of pigmentation, antiaging, dandruff and itching relaxation, hair loss reduction, prevention and remedy of skin diseases, storability improvement, softness improvement, elasticity improvement, glossiness impartment, melanin dye production inhibition, sunburn prevention, etc.

The ingredients that may be added to the skincare preparation for external use of the invention include, for example, oil and fat, phospholipid, UV absorbent, IR absorbent, emulsifier, surfactant, preservative, antifungal, antioxidant, skin whitener, vitamin, amino acid, hormone, peptide, physiologically-active vegetable extract, fluorescent material, pigment, dye, fragrance, scrub, metal ion sequestrant, binder, extender, tackifier, saccharide, nutrient composition, pH

EP 2 177 539 A1

controlling agent, chelating agent, microbicide, cuticle improver, keratolytic agent, antibiotic, skin penetration promoter, blood circulation promoter, antiphlogistic, cell activator, antiinflammatory agent, analgesic, skin softener, skin relaxing agent, sound remedy, metabolism promoter, etc. These may be added to the preparation in accordance with their use and object. In addition, any other moisturizing ingredient than the propylene glycol hyaluronate of the invention may be further added to the preparation.

Examples of the oil and fat ingredient that may be used in the skincare preparation for external use of the invention include fatty acids (e.g., oleic acid, behenic acid, isostearic acid, lauric acid, myristic acid, palmitic acid, stearic acid, behenic acid, linolic acid, γ-linolenic acid, columbic acid, eicosa-(n-6,9,13)-trienoic acid, arachidonic acid, α-linoleic acid, tymnodonic acid, hexanoic acid), ester oils (e.g., pentaerythritol tetra-2-ethylhexanoate, isopropyl myristate, butyl stearate, hexyl laurate, octyldodecyl myristate, diisopropyl adipate, diisopropyl sebacate), waxes (e.g., bees wax, spermaceti, lanolin, carnauba wax, candelilla wax, vaseline), animal oils and vegetable oils (e.g., mink oil, olive oil, castor oil, cacao butter, palm oil, cod liver oil, beef tallow, butter fat, evening primrose oil, rice bran oil, squalane), mineral oils (e.g., hydrocarbon oils, liquid paraffin), silicone oils (e.g., methylphenylsilicone, dimethylsilicone), higher alcohols (e.g., lauryl alcohol, stearyl alcohol, oleyl alcohol, cetyl alcohol, 2-octyldodecanol, 2-decyltetradecanol), and their derivatives. Also usable are organic acids such as α-hydroxy acid, hydroxycarboxylic acid, dicarboxylic acid, glycyrrhizic acid, glycyrrhetinic acid, mevalonic acid (mevalolactone), etc.

Examples of the phospholipid that may be used in the skincare preparation for external use of the invention include monoacyl ester-type glycerophospholipids and diacyl ester-type glycerophospholipids. Concretely, they include lysophosphatidylcholine, lysophosphatidylethanolamine, lysophosphatidylserine, lysophosphatidylinositol, phosphatidylcholine, phosphatidylethanolamine, phosphatidylinositol, phosphatidylserine, phosphatidylglycerol, phosphatidic acid, sphingomyelin. Naturally derived lecithins (e.g., egg yolk) and hydrogenated of the compounds mentioned above may also be used.

Examples of the UV absorbent that may be used in the skincare preparation for external use of the invention include oxybenzone (2-hydroxy-4-methoxybenzophenone), oxybenzonesulfonic acid, oxybenzonesulfonic acid (trihydrate), guaiazulene, ethylene glycol salicylate, octyl salicylate, dipropylene glycol salicylate, phenyl salicylate, homomenthyl salicylate, methyl salicylate, methyl diisopropylcinnamate, cinoxate (2-ethoxyethyl p-methoxycinnamate), glyceryl mono-2-ethylhexyl-di-p-methoxycinnamate, dihydroxymethoxybenzophenone, sodium dihydroxymethoxybenzophenonedisulfonate, dihydroxybenzophenone, tetrahydroxybenzophenone, p-aminobenzoic acid, ethyl p-aminobenzoate, glyceryl p-aminobenzoate, amyl p-dimethylaminobenzoate, 2-ethylhexyl p-dimethylaminobenzoate, p-hydroxyanisole, 2-ethylhexyl p-methoxycinnamate, isopropyl p-methoxycinnamate, diisopropyl cinnamate ester, 2-(2-hydroxy-5-methylphenyl)benzotriazole, sodium hydroxymethoxybenzophenonesulfonate, 4-tert-butyl-4'-methoxybenzoylmethane, 2-ethylhexyl salicylate, glyceryl p-monobenzoate, methyl orthoaminobenzoate, 2-hydroxy-4-methoxybenzophenone, amyl p-dimethylaminobenzoate, 2-phenylbenzimidazol-5-sulfonic acid, 2-hydroxy-4-methoxybenzophenone-5-sulfonic acid, dicaproyl trioleate, 2-ethoxyethyl p-methoxycinnamate, butylmethoxy-dibenzoylmethane, glyceryl mono-2-ethylhexanoyl-di-p-methoxybenzophenone, 2-ethylhexyl-2-cyano-3,3'-diphenylacrylate, 2,2'-dihydroxy-4-methoxybenzophenone, ethyl 4-bishydroxypropyl aminobenzoate.

The emulsifier and the surfactant that may be used in the skincare preparation for external use of the invention include nonionic surfactant, anionic surfactant, cationic surfactant and ampholytic surfactant. Examples of the nonionic surfactant include sorbitan esters (e.g., sorbitan monolaurate, sorbitan monooleate, sorbitan monoisostearate), polyoxyethylene sorbitan esters (e.g., polyoxyethylene sorbitan monoisostearate, polyoxyethylene sorbitan monolaurate, polyoxyethylene sorbitan monooleate), glycerol ethers (e.g., glycerol monoisostearate, glycerol monomyristate), polyoxyethylene glycerol ethers (e.g., polyoxyethylene glycerol monoisostearate, polyoxyethylene glycerol monomyristate), polyglycerin fatty acid esters (e.g., diglyceryl monostearate, decaglyceryl decaisostearate, diglyceryl diisostearate), glycerin fatty acid esters (e.g., glyceryl monocaprate, glyceryl monolaurate, glyceryl monomyristate, glyceryl monopalmitate, glyceryl monooleate, glyceryl monostearate, glyceryl monolinoleate, glyceryl monoisostearate, glyceryl monodilinoleate, glyceryl monodicaprate), polyoxyethylene glycerin fatty acid esters (e.g., polyoxyethylene glyceryl monomyristate, polyoxyethylene glyceryl monooleate, polyoxyethylene glyceryl monostearate), polyoxyethylene branched alkyl ethers (e.g., polyoxyethylene octyldodecyl alcohol, polyoxyethylene-2-decyltetradecyl alcohol), polyoxyethylene alkyl ethers (e.g., polyoxyethylene oleyl alcohol ether, polyoxyethylene cetyl alcohol ether), polyoxyethylene hardened castor oil fatty acid esters (e.g., polyoxyethylene hardened castor oil, polyoxyethylene dihydrocholesterol ether, polyoxyethylene hardened castor oil isostearate), polyoxyethylene alkyl aryl ethers (e.g., polyoxyethylene octyl phenol ether), etc.

Examples of the anionic surfactant include salts of higher fatty acids (e.g., oleic acid, stearic acid, isostearic acid, palmitic acid, myristic acid, behenic acid) (e.g., diethanolamine salts, triethanolamine salts, amino acid salts, potassium salts, sodium salts), ether carboxylic acid alkali salts, N-acylamino acid salts, N-acyl sarcosinates, higher alkyl sulfonates. Examples of the cationic or ampholytic surfactant include alkyl quaternary ammonium salts, polyamines alkyl amine salts, etc.

Examples of powders usable in the skin preparation for external use of the invention include talc, kaolin, fuller earth, gum, starch, silica, silicic acid, aluminium silicate hydrate, chemically modified aluminium magnesium silicate, sodium polyacrylate, tetraalkyl aryl ammonium smectite, trialkyl aryl ammonium smectite, ethylene glycol monostearate, sodium carboxymethylcellulose, carboxyvinyl polymer, chalk, gummy matter, ethylene glycol monostearate, ethylene glycol distearate.

Examples of polyols usable in the skincare preparation for external use of the invention include glycerin; polyglycerins such as diglycerin, triglycerin, tetraglycerin; ethylene glycol, propylene glycol, 1,3-butylene glycol, 1,4-butylene glycol, dipropylene glycol, polyethylene glycol, sorbitol, erythritol, maltotriose, threitol, sucrose, glucose, maltose, multitose, fructose, xylitose.

Other materials usable in the skincare preparation for external use of the invention include vitamins (e.g., vitamin A, vitamin $B_1$, vitamin $B_2$, vitamin $B_6$, vitamin $B_{12}$, vitamin C, vitamin D, vitamin E, vitamin K), amino acids (e.g., proline, leucine, isoleucine, alanine, threonine, lysine, cysteine, arginine), hormones (e.g., estrogen, pregnenolone, adrenocortical hormone), peptides (e.g., keratin, collagen, elastin), saccharides (for example, as listed above for polyols), inorganic salts (e.g., sodium chloride, sodium hydrogencarbonate, sodium carbonate, borax, sodium sulfate, sodium sulfide, sodium thiosulfate, sodium sesquicarbonate, magnesium oxide, calcium carbonate, magnesium carbonate, potassium chloride, potassium sulfide), *Streptococcus themophilus* cultures, sterols (e.g., cholesterol, provitamin $D_3$, campesterol, stigmastanol, stigmasterol, 5-dihydrocholesterol, α-spinastenol, cholesterol fatty acid esters), sphingosines (e.g., sphingosine, dihydrosphingosine, phytosphingosine, dehydrosphingosine, dehydrophytosphingosine, sphingadienine), ceramides, pseudoceramides, saponins, chitin derivatives, oligosaccharides (e.g., maltose, xylobiose, isomaltose, lactose, sucrose, raffinose, maltotriose, xylotriose, maltotetraose, xylotetraose, maltopentaose, xylopentaose, maltohexaose, xylohexaose, maltoheptaose, xyloheptaose), acid mucopolysaccharides (e.g., hyaluronic acid, chondroitin sulfate, dermatan sulfate, heparin, heparan sulfate), yeast extracts.

The skincare preparation for external use of the invention may further comprise thickeners (e.g., carboxyvinyl polymers, carboxymethyl cellulose, polyvinyl alcohol, carrageenan, xanthan gum, pullulan, alginates, propylene glycol alginate, polyglutamic acid, gelatin), electrolytes (e.g., sodium chloride), skin-whitening agents (e.g., hydroquinone and its derivatives, allantoin, vitamin E derivatives, glycyrrhizin, L-ascorbic acid and its derivatives, kojic acid, ellagic acid and its derivatives, tranexamic acid, resorcinol and its derivatives, panthellenic acid derivatives, placenta extract, coix seed extract, green tee, kudzu root, mulberry bark, licorice, scutellaria root, aloe, bitter orange peel, chamomile, *Ganoderma lucidum*), skin protective agents (e.g., retinol, retinol esters, retinoic acid, especially preferably water-soluble skin-whitening agents), skin softeners (e.g., stearyl alcohol, glyceryl monoricinoleate, mink oil, cetyl alcohol, stearic acid, coconut oil, castor oil, isostearic acid), emollients (e.g., stearyl alcohol, glycerin monoricinoleate, glycerin monostearate, cetyl alcohol), skin penetration enhancers (e.g., 2-methylpropane-2-ol, 2-propanol, ethyl 2-hydroxypropionate, 2,5-hexanediol, acetone, tetrahydrofuran), bioactive plant extracts (e.g., herb extracts of aloe, arnica, glycyrrhiza, sage, swertia, etc.), preservatives (e.g., p-hydroxybenzoate esters, sodium benzoate, urea, methylparaben, ethylparaben, propylparaben, butylparaben), antiinflammatory agents (e.g., salicylic acid), microbicides (e.g., triclosan), antioxidants (e.g., α-tocopherol, butylhydroxytoluene), buffers (e.g., triethanolamine or a combination of sodium hydroxide and lactic acid), keratolytic agents (e.g., lactic acid, glycolic acid, malic acid, tartaric acid, citric acid), scrubbing agents (e.g., polyethylene powder), pigments (e.g., calcium, barium or aluminium lake, iron oxide, titanium dioxide, mica, etc. The skincare preparation for external use of the invention may further comprise any other material than those mentioned above, depending on the use thereof. The amount of the ingredients to be added and the method for adding them can be determined according to the methods well known in the art.

EXAMPLES

The invention is described more concretely with reference to the following Examples and Test Examples. In the following Examples, the material and the reagent used, their ratio, and the details of the treatment may be suitably modified or changed not overstepping the sprit and the scope of the invention. Accordingly, the invention should not be limited to the Examples mentioned below.

(Example 1) Production of Propylene Glycol Hyaluronates

Sodium hyaluronate (300 g; FCH-200 available from Kibun Food Chemifa Co., Ltd.) was mixed with a mixed solution of 3.6 L of ethanol/water (9/1) and 180 ml of hydrochloric acid, and the mixture was stirred at 65°C for 3 hours. Next, this was washed with ethanol/water (9/1) to give a mixture of hyaluronic acid and sodium hyaluronate. 100 g (dry weight) of the mixture was mixed with 6 molar times of propylene oxide added thereto, and esterified at 70°C for 20 minutes. Next, this was washed with ethanol/water (9/1), neutralized with 5 moles of sodium acetate in ethanol/water (8.5/1.5) solution, and then washed with ethanol/water (9/1). The resulting reaction product was dried under reduced pressure to give propylene glycol hyaluronate. The esterification degree of the propylene glycol hyaluronate was 25.9%, and the limiting viscosity thereof was 1.2 dL/g (sample No. 1).

Different types of propylene glycol hyaluronates having a different esterification degree and a different limiting viscosity were produced in the same manner as above but changing the reaction condition (samples Nos. 2 to 5, 7 to 10). The analytic data of these propylene glycol hyaluronates are shown in Table 1. The esterification degree was determined in a test carried out according to IOB METHOD OF ANALYSIS 7.2 Determination of esterification degree. Samples Nos. 6 and 11 are sodium hyaluronate.

[Table 1]

| Sample No. | Limiting Viscosity (dL/g) | Esterification degree (%) |
|---|---|---|
| 1 | 1.2 | 25.9 |
| 2 | 6.3 | 36.0 |
| 3 | 15.1 | 21.7 |
| 4 | 0.2 | 56.0 |
| 5 | 0.5 | 52.0 |
| 6 | 0.3 | 0.0 |
| 7 | 0.5 | 72.6 |
| 8 | 2.8 | 59.7 |
| 9 | 2.3 | 65.5 |
| 10 | 0.5 | 58.4 |
| 11 | 4.5 | 0.0 |

(Test Example 1) Test for Arbutin Percutaneous Absorption Enhancement

Any of arbutin (Nippon Fine Chemical, 1%) and samples Nos. 1 to 9 (0.5%) was dissolved in an isotonic phosphate buffer (hereinafter referred to as "Km/PBS") having a pH of 7.1 and containing kanamycin (Wako Pure Chemical Industry, 0.01%) thereby to prepare sample solutions. A skin was prepared according to Fujii et al's method (Biol. Pharm. Bull., 20(3), 249. 1997). Briefly, a pig skin frozen and stored at -80°C (Yucatan Micropig Skin Set, Nippon Charles River) was spontaneously thawed, the excessive fat layer was removed, and the resulting skin was cut into pieces of about 3 cm square, and these were used herein. Next, the receptor of a membrane penetration test device (Vidrex's TP-8S) was filled with Km/PBS, and then the pig skin was set on it with its surface layer kept upside. Next, 2 ml of a sample solution was fed to the donor side, covered with a glass ball so as to protect it from vaporization, and this was kept at 37°C with stirring. 6 hours, 9 hours and 12 hours after the start of the stirring, the penetrated liquid was sampled in an amount of 400 μl, and every time at the sampling, 400 μl of Km/PBS was supplied to the device. The sampled liquid was analyzed through high-performance liquid chromatography. As a blank test, Km/PBS was supplied to the donor side; and as a control group, Km/PBS containing 1% arbutin was to it.

Condition of High-Performance Liquid Chromatography Apparatus

Pump:                L-7100 (by Hitachi High-Technologies)
UV Detector:         L-7400 (by Hitachi High-Technologies)
Column Oven:         L-7300 (by Hitachi High-Technologies)
Interface:           D-7000
Column:              TSK-gel ODS-120T, 150 mm × 4.6 mm (Tosoh)
Column Temperature:  40°C
Eluent:              aqueous 0.1% phosphoric acid solution/methanol (97/3)
Detection Wavelength: 285 nm
Flow Rate:           0.8 ml/min
Sample Amount:       20 μl

The samples were evaluated as follows: The accumulated penetration amount of each sample per cm of the skin piece was determined, and this was converted according to the following formula:

```
[Numerical Formula 1]
Percutaneous Absorption Enhancing Effect
= (accumulated penetration amount of each sample)/(accumulated
penetration amount of control sample)
```

[Table 2]

| Sample No. | Percutaneous Absorption Enhancing Effect | | |
|---|---|---|---|
| | 6 hours | 9 hours | 12 hours |
| Control | 1.00 | 1.00 | 1.00 |
| 1 | - | - | 2.78 |
| 2 | - | - | 0.22 |
| 3 | - | - | 0.17 |
| 4 | 0.63 | 0.62 | 0.93 |
| 5 | 4.78 | 3.35 | 3.14 |
| 6 | 0.94 | 0.84 | 0.91 |
| 7 | 3.79 | 2.48 | 2.14 |
| 8 | 0.88 | 0.57 | 0.64 |
| 9 | 1.00 | 0.80 | 0.87 |

From the results in Table 2, it has been confirmed that samples Nos. 1, 5 and 7 especially have a remarkable percutaneous absorption-enhancing effect.

(Test Example 2) Test for Kojic Acid Percutaneous Absorption Enhancement

The same percutaneous absorption enhancement test as above was carried out, in which, however, arbutin in Test Example 1 was changed to kojic acid (by Wako Pure Chemical Industry) . Like in Test Example 1, the penetration amount after 12 hours was measured. As a result, the sample No. 5 showed koj ic acid penetration of 6. 6 times, as compared with the sample No. 2, and this confirms an especially remarkable percutaneous absorption enhancing effect of the sample. In addition, it has also been confirmed that the sample No. 1 and the sample No. 7 also show higher kojic acid penetration than the sample No. 2.

(Test Example 3) Test for Ascorbic Acid Derivative Percutaneous Absorption Enhancement

The same percutaneous absorption enhancement test as above was carried out, in which, however, arbutin in Test Example 1 was changed to magnesium ascorbate phosphate (by Wako Pure Chemical Industry). Like in Test Example 1, the penetration amount after 12 hours was measured. As a result, the sample No. 5 showed magnesium ascorbate phosphate penetration of 7.5 times, as compared with the sample No. 2, and this confirms an especially remarkable percutaneous absorption enhancing effect of the sample. In addition, it has also been confirmed that the sample No. 1 and the sample No. 7 also show higher magnesium ascorbate phosphate penetration than the sample No. 2.

(Test Example 4) Test for Oxygen Resistance

Samples Nos. 1 to 9 were individually dissolved in a phosphoric acid buffer (pH 6) so that the sample content thereof could be 0.1%, thereby preparing test liquids heated at 60˚C. Endo-hyaluronidase (Amano Enzyme's Ec 4.2.2.1 Hyaluronate Lyase) dissolved in physiological saline water was added to the test liquid in an amount of 1 TRU (turbidity reducing unit). After 90 minutes, the light absorbance at a wavelength of 230 nm of the sample was measured.

As a result, it has been confirmed that samples No. 2, No. 3 and No. 6 had a large absorbance, therefore having low oxygen resistance, while the other samples all had high oxygen resistance.

(Test Example 5) Test for Water Content in Horny Layer

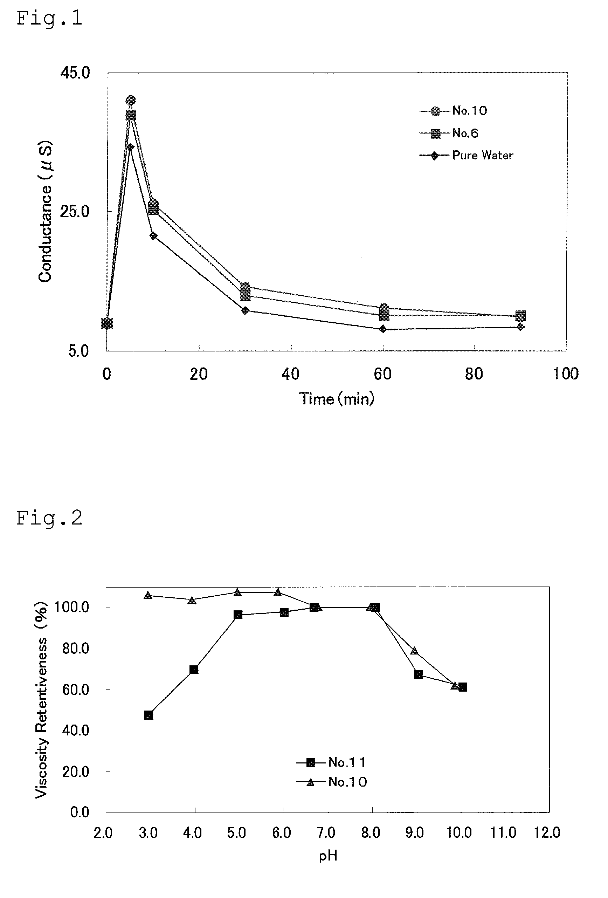

An aqueous 1.0% solution of the sample No. 10 was applied to the skin of 10 panelists, and sealed up for 1 hour. In a room kept at predetermined temperature and humidity, the conductance of the horn layer just before the application and 90 minutes after the application was measured. The sample No. 6 and pure water were tested in the same manner. The results are shown in Fig. 1.

The results in Fig. 1 confirm that the water content of the horny layer to which the sample No. 10 of the invention had been applied was higher than that to which pure water or sodium hyaluronate (sample No. 6) had been applied.

(Test Example 6) Test 1 for Stringiness

0.1 g of any of the samples Nos. 1 to 5 was dissolved in 10 ml of water to prepare an aqueous 1% solution thereof. An iron bar having a diameter of 5.5 mm was previously dipped in the solution by 1 cm, and the beaker was made to descend at a speed of 50 mm/min, whereupon the time taken before the liquid leaved the iron bar after the tip of the iron bar reached the liquid surface was measured, and the length of stringiness was measured. One sample was tested 10 times in the same test, and the data were averaged. The average is the length of stringiness of the tested sample. As a control, water was tested in the same test.

Table 3 shows the length of stringiness of each sample and control.

[Table 3]

| Sample No. | Mean Length of Stringiness (mm) |
|---|---|
| Control | 3.12 |
| 1 | 2.90 |
| 2 | 3.34 |
| 3 | 5.28 |
| 4 | 3.10 |
| 5 | 3.07 |

The results in Table 3 confirm that the mean length of stringiness of the samples No. 1, No. 4 and No. 5 is on the same level as that of the control. Samples with poorer stringiness are advantageous in that they are easy to handle and their feel is good.

(Test Example 7) Test 2 for Stringiness

Aqueous 2% solution and aqueous 5% solution of the sample No. 10 were prepared. Aqueous 2% solution and aqueous 5% solution of the sample No. 11 were also prepared. These aqueous solutions were tested according to the method of Test Example 6 to determine the mean length of stringiness thereof. As a control, water was tested in the same test.

[Table 4]

| Sample No. | Mean Length of Stringiness (mm) | |
|---|---|---|
| | Aqueous 2% Solution | Aqueous 5% Solution |
| Control | 3.20 | 3.20 |
| 10 | 3.11 | 3.09 |

(continued)

| Sample No. | Mean Length of Stringiness (mm) | |
|---|---|---|
| | Aqueous 2% Solution | Aqueous 5% Solution |
| 11 | 3.33 | 4.19 |

The results in Table 4 confirm that the sample No. 10 of the invention has poorer stringiness, therefore depressing the surface tension of water. On the other hand, the stringiness of the sample No. 11, sodium hyaluronate was relatively high.

(Test Example 8) Hydration Test

Using the samples Nos. 1 to 5, aqueous 0.2%, 0.1% and 0.05% solutions were prepared each in an amount of 50 ml. A filter paper was dipped in the aqueous solution by 1 cm from its edge, and kept as such for 30 minutes. Then, the filter paper was pulled up from the aqueous solution, and the moving distance from the liquid surface (liquid mobility) was measured. The results are shown in Table 5.
[Table 5]

| Sample | Water Mobility (mm) | | |
|---|---|---|---|
| | 0.05% | 0.10% | 0.20% |
| 1 | 83 | 69 | 63 |
| 2 | 42 | 32 | 29 |
| 3 | 23 | 19 | 11 |
| 4 | 94 | 94 | 88 |
| 5 | 93 | 86 | 80 |

The results in Table 5 confirm that the mobility with the samples No. 1, No. 4 and No. 5 is large and that the hydration of these samples is low. Samples with lower hydration have the advantage in that they hardly flow away with sweat when applied to a skin.

(Test Example 9) Test 1 for Solubility in Alcohol

The samples Nos. 1 to 5 were sampled each in an amount of 0.1 g. 10 ml of 90% ethanol, 80% ethanol, 75% ethanol, 70% ethanol, 60% ethanol, 100% glycerin, 80% glycerin or 40% glycerin was added to each sample. After left overnight at 25˚C, the solubility of each sample was evaluated as four ranks of ◎, ○, △ and ×.
The solubility of the samples is shown in Table 6.
[Table 6]

| Sample No. | Ethanol | | | | | Glycerin | | |
|---|---|---|---|---|---|---|---|---|
| | 90% | 80% | 75% | 70% | 60% | 100% | 80% | 40% |
| 1 | × | × | × | ◎ | ◎ | × | ◎ | ◎ |
| 2 | × | × | × | ○ | ◎ | × | ○ | ◎ |
| 3 | × | × | × | △ | ◎ | × | ○ | ◎ |
| 4 | × | △ | ○ | ◎ | ◎ | × | ◎ | ◎ |

(continued)

| Sample No. | Ethanol | | | | | Glycerin | | |
|---|---|---|---|---|---|---|---|---|
| | 90% | 80% | 75% | 70% | 60% | 100% | 80% | 40% |
| 5 | × | × | ○ | ◎ | ◎ | × | ◎ | ◎ |

◎: Completely transparent.
○: Almost dissolved.
△: Partly dissolved.
×: Cloudy or precipitate deposition.

The results in Table 6 confirm that the samples No. 1, No. 4 and No. 5 dissolved relatively easily in an alcohol solvent. Easy dissolution in solvent is advantageous in that the sample is easy to handle and is readily applicable to various use typically for cosmetics.

(Test Example 10) Test 2 for Solubility in Alcohol

Ethanol was added to an aqueous 0.1% solution of the sample No. 10 so that the final ethanol solution in the resulting solutions could be 90%, 85%, 80%, 70%, 60% or 50%. Similarly, glycerin was added to the solution so that the final glycerin concentration therein could be 100%, 80% or 40%. These were left overnight at 25°C and the solubility was evaluated as four ranks of ◎, ○, △ and ×. The sample No. 11 was tested in the same manner.
Table 7 shows the solubility of the samples.
[Table 7]

| Sample No. | Ethanol | | | | | | | glycerin | | |
|---|---|---|---|---|---|---|---|---|---|---|
| | 95% | 90% | 85% | 80% | 70% | 60% | 50% | 100% | 80% | 40% |
| 10 | △ | ○ | ◎ | ◎ | ◎ | ◎ | ◎ | × | ◎ | ◎ |
| 11 | × | × | × | ○ | ○ | ◎ | ◎ | × | ◎ | ◎ |

◎: Completely transparent.
○: Almost dissolved.
△: Partly dissolved.
×: Cloudy or precipitate deposition.

The results in Table 7 confirm that the sample No. 10 of the invention is more soluble in the alcohol solvents than sodium hyaluronate (sample No. 11). They also confirm that the sample No. 10 of the invention may dissolve in the solvents except 100% glycerin within a short period of time. Easy dissolution in solvent is advantageous in that the sample is easy to handle and is readily applicable to various use typically for cosmetics.

(Test Example 11) Test for Cationic Substance Dissolution

An aqueous chitosan solution was added to an aqueous solution comprising each sample so as to have a final chitosan concentration of 0.1%, and the sample dissolution was determined.
In addition, a mixture of chitosan (final concentration 0.1%) and polyoxyethylene(20) sorbitan monooleate (1.0%); a mixture of lauryltrimethylammonium chloride (1.0%), betaine lauryldimethylaminoacetate (1.0%) and lauryltrimethylammonium chloride (1.0%); a mixture of polyoxyethylene(20) sorbitan monooleate (1.0%) and lauryltrimethylammonium chloride (1.0%) were also tested for their solubility. Chitosan was Katakura Chikkarin's CTF; polyoxyethylene(20) sorbitan monooleate was Kao's Rheodol Super TW-120; lauryltrimethylammonium chloride was Kao's Quartamin 24P; and betaine lauryldimethylaminoacetate was Kao's Amphitol 24B.
As a result, the samples No. 2 and No. 3 became cloudy and formed precipitate; and their solubility was extremely poor. The solubility of the sample No. 6 was not also good. As opposed to these, the samples No. 5 and No. 7 did not form precipitate, and had excellent solubility. In particular, they gave transparent liquid with a combination of a cationic substance and a nonionic substance.

(Test Example 12) Test for Viscosity Stability

Aqueous 1% solutions were prepared with each of the samples No. 10 and No. 11, and their viscosity was measured within a pH range of from 3 to 10. The viscosity at pH 7 was taken as 100; and the viscosity at other pH was represented by a relative value to it. The viscosity data are shown in Fig. 2.
The results in Fig. 2 confirm that the viscosity of sodium hyaluronate lowers especially in an acid region; but the sample No. 10 of the invention keeps good viscosity stability in any region including an acidic region.

(Test Example 13) Test for Emulsification

Aqueous 1% and 0.2% solutions of the samples Nos. 1 to 5 were prepared. Liquid paraffin (Kaneda's Hicall K-350) was mixed with each solution in a ratio of 1/1. With heating in a constant-temperature hot water tank at 70°C, this was stirred at 5000 rpm for 1 minute, using a hand homogenizer, and then, this was taken out of the tank and further stirred at 30000 rpm for 1 minute. After statically left for 1 hour, this was checked for its condition phase separation, and the size of the emulsified droplets in each sample was comparatively determined.
As a result, all the samples showed good emulsification.

(Test Example 14) Test for Emulsion Stability

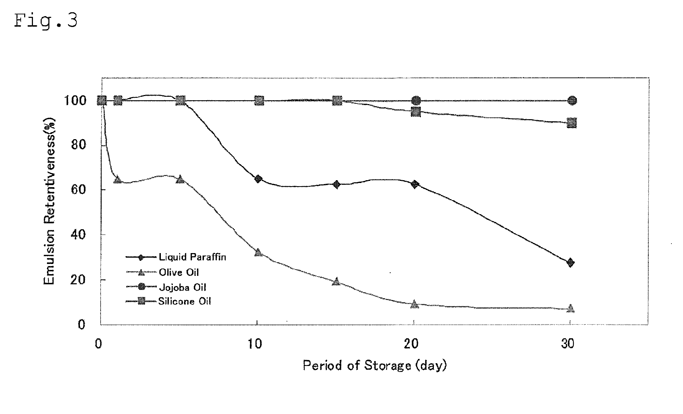

An aqueous 0.5% solution of the sample No. 10 was prepared; and any of liquid paraffin, olive oil, jojoba oil or silicone oil was added thereto to have an oil concentration of 70%. With heating in a constant-temperature hot water tank at 70°C for 1 minute, this was stirred at 5000 rpm for 1 minute, using a hand homogenizer, and then, this was taken out of the tank and further stirred at 30000 rpm for 1 minute. This was statically left at 50°C, and its emulsion retentiveness for 30 days was determined. The emulsion retentiveness as referred to herein is represented by the ratio of the emulsion phase after left for a period of time, relative to the thickness of the emulsion phase before the storage taken as 100. The results are shown in Fig. 3.
On the other hand, sodium hyaluronate of the sample No. 11 was tested in the same manner. This was completely separated into an aqueous phase and an oily phase after 1 day, and its emulsion stability was poor.
The results in Fig. 3 confirm that the sample No. 10 of the invention has an excellent emulsifying effect for liquid paraffin, olive oil, jojoba oil and silicone oil, as compared with sodium hyaluronate.

(Test Example 15) Test for Feel in Use

An aqueous 0.1% solution of each of the samples Nos. 1 to 5 was prepared. Women panelists (twenties to fifties, 6 in all) and men panelists (twenties to fifties, 7 in all), totaling 13 panelists, tested every sample. Each sample was applied to the back of the hand or to the forearm of every panelist, who checked it in point of the freshness and the spreadability for 7 ranks of from -3 to +3. The mean score of each test is shown in Table 8.
[Table 8]

| Sample No. | Freshness | Spreadability |
|------------|-----------|---------------|
| 1 | 2.1 | 0.6 |
| 2 | 1.9 | 0.2 |
| 3 | 0.1 | 0.1 |
| 4 | 1.8 | 0.8 |
| 5 | 1.5 | 0.5 |

Samples having a score of at least 1 for the freshness and having a score of at least 0.4 for the spreadability are especially good samples. The results in Table 8 confirm that the samples No. 1, No. 4 and No. 5 are especially good.

(Example 2) Production of Beauty Lotions

Various ingredients shown in the table below were mixed at room temperature and thoroughly stirred to prepare beauty lotions. In the following examples, the "active ingredient" refers to a propylene glycol hyaluronate having an esterification degree of from 10 to 90% and a limiting viscosity of less than 3 L/g.

[Table 9]

| Ingredients | part by weight |
|---|---|
| Active Ingredient | 1.0 |
| Methylparaben | 0.1 |
| Polyoxyethylene Hardened Caster Oil | 1.2 |
| Polyoxyethylene Sorbitan Oleate | 0.4 |
| Ethanol | 5.3 |
| Pure Water | 92.0 |

(Example 3) Production of Powder Foundations

Various ingredients shown in the table below were mixed at room temperature and thoroughly stirred to prepare powder foundations.

[Table 10]

| Ingredients | part by weight |
|---|---|
| Active Ingredient | 1.0 |
| Mica | 37.8 |
| Talc | 20.0 |
| Titanium Dioxide | 12.0 |
| Kaolin | 5.0 |
| Iron Oxide | 3.5 |
| Powdery Nylon | 8.0 |
| Octyldodecyl Myristate | 2.0 |
| Neopentyl Glycol Diisooctanoate | 2.0 |
| Sorbitan Monooleate | 0.5 |
| Zinc Stearate | 1.0 |
| Red Iron Oxide | 1.0 |
| Squalane | 6.0 |
| Preservative | 0.1 |
| Antioxidant | 0.1 |

(Example 4) Production of Skin-Whitening Powders

Various ingredients shown in the table below were mixed and powdered at room temperature to prepare skin-whitening powders.

[Table 11]

| Ingredients | part by weight |
|---|---|
| Active Ingredient | 20.0 |

(continued)

| Ingredients | part by weight |
|---|---|
| Sucrose | 50.0 |
| Polyethylene Glycol | 10.0 |
| Silica | 4.5 |
| Vitamin C | 5.0 |
| Vitamin C Dipalmitate | 10.0 |
| Colorant | 0.5 |

(Example 5) Production of Emollient Creams

After 1,3-butylene glycol and pure water shown in the table below were mixed and heated to 70˚C, a mixture of the remaining ingredients molten by heating was added and the emulsified particles were homogenized and cooled to prepare emollient creams.
[Table 12]

| Ingredients | part by weight |
|---|---|
| Active Ingredient | 5.0 |
| Stearyl Alcohol | 6.0 |
| Stearic Acid | 2.0 |
| Hydrogenated Lanolin | 4.0 |
| Squalane | 9.0 |
| Octyl Dodecanol | 10.0 |
| POE(25) Cetyl Alcohol Ether | 3.0 |
| Glycerin Monostearate | 2.0 |
| 1,3-Butylene Glycol | 10.0 |
| Colorant | 0.5 |
| Preservative | 0.1 |
| Antioxidant | 0.1 |
| Pure Water | 48.3 |

(Example 6) Production of Cleansing Foams

Stearic acid, palmitic acid, myristic acid, lauric acid, coconut oil and preservative shown in the table below were melted by heating and kept at 70˚C, and a mixture of potassium hydroxide and pure water was added thereto with stirring. Then, the remaining ingredients were added and the mixture was thoroughly stirred and then degassed and cooled to prepare cleansing foams.
[Table 13]

| Ingredients | part by weight |
|---|---|
| Active Ingredient | 4.5 |
| Stearic Acid | 10.0 |
| Palmitic Acid | 10.0 |

(continued)

| Ingredients | part by weight |
|---|---|
| Myristic Acid | 12.0 |
| Lauric Acid | 4.0 |
| Coconut Oil | 2.0 |
| Potassium Hydroxide | 6.0 |
| Glycerol Monostearate Ester | 2.0 |
| POE(20) Sorbitan Monostearate | 2.0 |
| Colorant | 0.5 |
| Preservative | 0.1 |
| Chelating Agent | 0.2 |
| Pure Water | 46.7 |

(Example 7) Production of Packs

Titanium oxide and talc shown in the table below were thoroughly dispersed in pure water, then sorbitol was added thereto and dissolved under heat at 70˚C. Then, the remaining ingredients were added thereto and fully stirred, and degassed and cooled to prepare pasty packs.
[Table 14]

| Ingredients | part by weight |
|---|---|
| Active Ingredient | 4.5 |
| Polyvinyl Acetate Emulsion | 15.0 |
| Polyvinyl Alcohol | 10.0 |
| Jojoba Oil | 2.0 |
| Squalane | 2.0 |
| POE Sorbitan Monostearate Ester | 1.0 |
| Titanium Oxide | 5.0 |
| Talc | 10.0 |
| Sorbitol | 10.0 |
| Ethanol | 8.0 |
| Colorant | 0.5 |
| Preservative | 0.2 |
| Pure Water | 31.8 |

(Example 8) Production of Lipsticks

Various ingredients shown in the table below were heated at 70˚C, and then mixed. The mixture was thoroughly stirred and cast and then rapidly cooled to prepare lipsticks.
[Table 15]

| Ingredients | part by weight |
|---|---|
| Active Ingredient | 2.0 |

(continued)

| Ingredients | part by weight |
|---|---|
| Castor Oil | 25.0 |
| Cetyl 2-Ethylhexanoate | 20.0 |
| Lanolin | 10.0 |
| Isopropyl Myristate Ester | 10.0 |
| Candelilla Wax | 9.0 |
| Solid Paraffin | 8.0 |
| Carnauba Wax | 5.0 |
| Bees Wax | 5.0 |
| Titanium Dioxide | 5.0 |
| Colorant | 1.0 |

(Example 9) Production of Lip Creams

Active ingredient, stearic acid, stearyl alcohol and butyl stearate shown in the table below were heated at 70°C and then mixed, and a mixture of the remaining ingredients was added thereto and fully stirred to prepare lip creams.
[Table 16]

| Ingredients | part by weight |
|---|---|
| Active Ingredient | 4.0 |
| Stearic Acid | 14.0 |
| Stearyl Alcohol | 8.0 |
| Butyl Stearate | 10.0 |
| Propylene Glycol | 10.0 |
| Glycerin Monostearate | 4.0 |
| Potassium Hydroxide | 1.0 |
| Antioxidant | 0.2 |
| Pure Water | 48.8 |

(Example 10) Production of Cheek Colors

Other ingredients than fragrance and liquid paraffin shown in the table below were mixed at room temperature and then sprayed with fragrance and liquid paraffin and pulverized. The mixture was compression-molded to prepare cheek colors.
[Table 17]

| Ingredients | part by weight |
|---|---|
| Active Ingredient | 1.5 |
| Talc | 77.8 |
| Kaolin | 9.0 |
| Zinc Myristate | 5.0 |
| Pigment | 3.0 |

(continued)

| Ingredients | part by weight |
|---|---|
| Liquid Paraffin | 3.0 |
| Fragrance | 0.5 |
| Preservative | 0.2 |

(Example 11) Production of Eyeliners

Carbon black shown in the table below was pulverized and then dispersed in pure water, with which the remaining ingredients were mixed at room temperature to prepare eyeliners.
[Table 18]

| Ingredients | part by weight |
|---|---|
| Active Ingredient | 10.0 |
| Carbon Black | 5.0 |
| Polyoxyethylene Dodecyl Ether | 2.0 |
| Colorant | 0.5 |
| Preservative | 0.2 |
| Pure Water | 82.3 |

(Example 12) Production of Mascaras

Iron oxide, pure water and polyacrylate ester emulsion shown in the table below were mixed at 70˚C, then a mixture prepared by heating and melting the remaining ingredients at 70˚C was added to it and emulsified and dispersed to prepare mascaras.
[Table 19]

| Ingredients | part by weight |
|---|---|
| Active Ingredient | 4.5 |
| Iron Oxide | 10.0 |
| Polyacrylate Ester Emulsion | 27.0 |
| Solid Paraffin | 8.0 |
| Lanolin Wax | 8.0 |
| Light Isoparaffin | 28.0 |
| Sorbitan Sesquioleate | 4.0 |
| Colorant | 0.5 |
| Antioxidant | 0.1 |
| Preservative | 0.1 |
| Pure Water | 9.8 |

(Example 13) Production of Eyebrow Colors

Other ingredients than the powdery ingredients shown in the table below were molten and mixed, and then the powdery ingredients were added thereto, kneaded and molded to prepare eyebrow colors.

[Table 20]

| Ingredients | part by weight |
|---|---|
| Active Ingredient | 1.0 |
| Iron Oxide | 19.0 |
| Titanium Oxide | 5.0 |
| Talc | 10.0 |
| Kaolin | 15.0 |
| Japan Wax | 20.0 |
| Stearic Acid | 10.0 |
| Bees Wax | 5.0 |
| Hardened Castor Oil | 5.0 |
| Vaseline | 4.0 |
| Lanolin | 3.0 |
| Liquid Paraffin | 2.8 |
| Antioxidant | 0.1 |
| Preservative | 0.1 |

(Example 14) Production of Hand Creams

Various ingredients shown in the table below were mixed under heat at 70˚C and thoroughly stirred to prepare hand creams.

[Table 21]

| Ingredients | part by weight |
|---|---|
| Active Ingredient | 3.0 |
| Glycerin | 20.0 |
| Urea | 2.0 |
| Stearic Acid Monoglyceride | 2.5 |
| Vaseline | 6.0 |
| Liquid Paraffin | 10.0 |
| Pure Water | 56.5 |

(Example 15) Production of Hair Shampoos

Various ingredients shown in the table below were mixed under heat at 70˚C and thoroughly stirred to prepare hair shampoos.

[Table 22]

| Ingredients | part by weight |
|---|---|
| Active Ingredient | 5.0 |
| Glycerin | 1.0 |

(continued)

| Ingredients | part by weight |
|---|---|
| Sodium Lauryl Polyoxyethylene Sulfate | 10.0 |
| Sodium Laurylsulfate | 6.0 |
| Coconut Oil Fatty Acid Diethanolamide | 3.0 |
| Metal Ion Sequestrant | 0.1 |
| pH-Controlling Agent | 0.5 |
| Preservative | 0.2 |
| Pure Water | 74.2 |

(Example 16) Production of Hair Rinses

Various ingredients shown in the table below were mixed under heat at 70˚C, and thoroughly stirred to prepare hair rinses.
[Table 23]

| Ingredients | part by weight |
|---|---|
| Active Ingredient | 3.0 |
| Silicone Oil | 2.8 |
| Liquid Paraffin | 1.2 |
| Glycerin | 2.5 |
| Cetyl Alcohol | 1.3 |
| Stearyl Alcohol | 1.1 |
| Stearyltrimethylammonium Chloride | 0.6 |
| Colorant | 1.0 |
| Preservative | 0.2 |
| Pure Water | 86.3 |

(Example 17) Production of Hair Liquids

Various ingredients shown in the table below were mixed at room temperature to prepare hair liquids.
[Table 24]

| Ingredients | part by weight |
|---|---|
| Active Ingredient | 1.0 |
| Polyoxypropylene Butyl Ether | 20.0 |
| Polyoxyethylene Hardened Castor Oil | 1.0 |
| Ethanol | 50.0 |
| Fragrance | 0.5 |
| Pure water | 27.5 |

(Example 18) Production of Bath Formulas

Various ingredients shown in the table below were mixed at room temperature to prepare bath formulas.
[Table 25]

| Ingredients | part by weight |
| --- | --- |
| Active Ingredient | 10 |
| Sodium Sulfate | 50 |
| Sodium Hydrogencarbonate | 25 |
| Sodium Chloride | 13 |
| Colorant | 2 |

(Example 19) Production of Skin-Whitening Creams

Of the ingredients shown in the table below, the aqueous phase was added to the oily phase, both heated and dissolved at 70°C, and then processed with a homogenizer and cooled to prepare skin-whitening creams.
[Table 26]

| Ingredients | part by weight |
| --- | --- |
| Microcrystalline Wax | 8.0 |
| Paraffin | 2.0 |
| Bees Wax | 3.0 |
| Reduced Lanolin | 8.0 |
| Squalane | 34.0 |
| Vaseline | 5.0 |
| Hexadecyl Adipate Ester | 10.0 |
| Oleophilic Glycerin Monooleate | 3.5 |
| Polyoxyethylene Sorbitan Monooleate Ester (20 E.O.) | 1.0 |
| Fragrance | 0.5 |
| Preservative, Antioxidant | ad lib. |
| Glycerin | 2.0 |
| Active Ingredient | 0.5 |
| Arbutin | 1.0 |
| Pure Water | 21.5 |

BRIEF DESCRIPTION OF THE DRAWINGS

[Fig. 1] This is a graph showing the results of the test for water content in horny layer.
[Fig. 2] This is a graph showing the results of the test for pH-dependent viscosity stability.
[Fig. 3] This is a graph showing the results of the test for emulsion stability.

**Claims**

1. A propylene glycol hyaluronate having an esterification degree of from 10 to 90% and a limiting viscosity of less than 3 dL/g.

2. A propylene glycol hyaluronate having an esterification degree of from 10 to 90% and a limiting viscosity of from 0.3 to 2.0 dL/g.

3. A propylene glycol hyaluronate having an esterification degree of from 10 to 90% and a limiting viscosity of from 0.4 to 1.6 dL/g.

4. A propylene glycol hyaluronate having an esterification degree of from 20 to 80% and a limiting viscosity of less than 3 dL/g.

5. A propylene glycol hyaluronate having an esterification degree of from 20 to 80% and a limiting viscosity of from 0.3 to 2.0 dL/g.

6. A propylene glycol hyaluronate having an esterification degree of from 20 to 80% and a limiting viscosity of from 0.4 to 1.6 dL/g.

7. A propylene glycol hyaluronate having an esterification degree of from 25 to 73% and a limiting viscosity of less than 3 dL/g.

8. A propylene glycol hyaluronate having an esterification degree of from 25 to 73% and a limiting viscosity of from 0.3 to 2.0 dL/g.

9. A propylene glycol hyaluronate having an esterification degree of from 25 to 73% and a limiting viscosity of from 0.4 to 1.6 dL/g.

10. A composition comprising the propylene glycol hyaluronate of any one of claims 1 to 9 and a cosmetically or pharmaceutically active compound.

11. The composition according to claim 10, wherein the cosmetically or pharmaceutically active compound is a skin-whitening active compound.

12. A skincare preparation for external use comprising the propylene glycol hyaluronate of any one of claims 1 to 9 or the composition of claim 10 or 11.

13. A moisturizer comprising the propylene glycol hyaluronate of any one of claims 1 to 9 or the composition of claim 10 or 11.

14. An emulsion comprising the propylene glycol hyaluronate of any one of claims 1 to 9 or the composition of claim 10 or 11.

15. A skin whitener comprising the propylene glycol hyaluronate of any one of claims 1 to 9 and a skin-whitening active compound.

16. The skin whitener according to claim 15, wherein the skin-whitening active compound is arbutin, kojic acid or an ascorbic acid derivative.

Fig.1

Fig.2

Fig.3

## INTERNATIONAL SEARCH REPORT

| | International application No. |
|---|---|
| | PCT/JP2008/002079 |

A.   CLASSIFICATION OF SUBJECT MATTER
*C08B37/08*(2006.01)i, *A61K8/60*(2006.01)i, *A61K8/73*(2006.01)i, *A61K47/36*(2006.01)i, *A61Q1/00*(2006.01)i, *A61Q5/00*(2006.01)i, *A61Q19/00*(2006.01)i, *A61Q19/02*(2006.01)i, *B01F17/56*(2006.01)i
According to International Patent Classification (IPC) or to both national classification and IPC

B.   FIELDS SEARCHED

Minimum documentation searched (classification system followed by classification symbols)
C08B37/08, A61K8/60, A61K47/36, B01F17/56

Documentation searched other than minimum documentation to the extent that such documents are included in the fields searched
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
WPI

C.   DOCUMENTS CONSIDERED TO BE RELEVANT

| Category* | Citation of document, with indication, where appropriate, of the relevant passages | Relevant to claim No. |
|---|---|---|
| A | JP 2001-233901 A  (Kabushiki Kaisha Kibun Fudo Kemikaru),<br>28 August, 2001 (28.08.01),<br>& EP 1110971 A1        & US 2004/0013627 A1<br>& US 6710038 B1 | 1-15 |
| A | JP 62-64802 A  (Francesco della Valle),<br>23 March, 1987 (23.03.87),<br>& EP 696598 A1        & US 4851521 A<br>& KR 10-1987-0001901 B1 | 1-15 |
| A | JP 58-183938 A  (Shiseido Co., Ltd.),<br>27 October, 1983 (27.10.83),<br>(Family: none) | 1-15 |

☐ Further documents are listed in the continuation of Box C.        ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered   to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 20 October, 2008 (20.10.08) | 28 October, 2008 (28.10.08) |

| Name and mailing address of the ISA/ | Authorized officer |
|---|---|
| Japanese Patent Office | |
| Facsimile No. | Telephone No. |

Form PCT/ISA/210 (second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001233901 A